# EUROPEAN PATENT SPECIFICATION

(11) **EP 0 323 141 B1**
(45) Date of publication and mention of the grant of the patent: **16.03.1994**
(21) Application number: 88312186.5
(22) Date of filing: 22.12.1988
(51) Int. Cl.: C07C 69/618, C07C 67/38

(54) **Preparation of cinnamate esters**
Herstellung von Zimtsäureestern
Préparation d'esters cinnamiques

(30) Priority: 28.12.1987 JP 330164/87
(43) Date of publication of application: 05.07.1989
(73) Proprietor: MITSUI TOATSU CHEMICALS, Inc., Chiyoda-Ku Tokyo 100 (JP)
(72) Inventor: Takaki, Usaji, Fujisawa-shi Kanagawa-ken (JP); Aoki, Shinobu 2-11, Mitsui, Sakae-ku Yokohama-shi Kanagawa-ken (JP); Yamamoto, Yoshihiro, Sakae-ku Yokohama-shi Kanagawa-ken (JP); Hara, Isao, Sakae-ku Yokohama-shi Kanagawa-ken (JP)
(74) Representative: West, Alan Harry

(56) References cited:
- EP-A- 0 177 354
- EP-A- 0 242 072

## Description

This invention relates to a process for preparing a cinnamate ester by reacting its corresponding styrene and alcohol, carbon monoxide and oxygen in the presence of a catalyst, and more specifically to a process for conducting the above reaction smoothly, and in particular to a method for removing heat evolved in a reactor in which the above reaction is conducted.

Cinnamate esters are used widely as perfume bases or starting materials therefor. They are also important as starting materials for agricultural chemicals and photosensitive resins and also for phenyl alanines as amino acids.

Cinnamic acid has conventionally been produced by the process in which benzaldehyde and an acetic acid derivative are used as principal starting materials.

This process however uses such expensive compounds as starting materials, so that the product becomes costly as a consequence. It is hence not a preferable process from the industrial standpoint. As a process making use of more inexpensive starting materials, it has been proposed to prepare a cinnamate ester by reacting a styrene in the presence of carbon monoxide, an alcohol, oxygen and a catalyst. Reference may be had, for example, to Japanese Patent Application Laid-Open Nos. 15242/1981, 70836/1982, 92242/1985 and 77352/1987.

The above reaction is, however, an oxidative reaction and its heat release value is extremely great. For example, it is calculated that the heat of reaction in converting styrene into methyl cinnamate is about 81 Kcal/mole. It is therefore extremely important to effect good heat removal in such a reaction. Routine heat removal procedures involve the use of external cooling jackets and internal cooling tubes for small scale operations, and external heat exchangers for larger scale or industrial scale operations. Alternatively, it is possible to utilise the cooling effects of evaporation and it is this latter procedure which is incorporated into the proposals described in EP-A-0177354 and EP-A-0242072. Both of those documents describe the catalytic preparation of cinnamate esters from the corresponding styrene and alcohol, carbon monoxide and oxygen and involve the condensation of vapours removed from the reaction mixture by evaporation, and return of the condensate to the reaction mixture. Those procedures involve also the addition of CO₂ or an inert gas to the reaction system in order to optimise the reaction conditions, including heat dissipation.

The present invention is based on the observation that the rates of evaporation of the liquid components in the reaction mixture and hence the rate of cooling of the reaction mixture can be enhanced by blowing into the reaction mixture through a separate feed port a gas selected from a portion of the gas discharged from the reaction, an inert gas and carbon dioxide.

In accordance with the invention, there is now provided a process for preparing a cinnamate ester which comprises reacting the corresponding styrene compound with an alcohol, carbon monoxide and oxygen in the presence of a catalyst containing palladium metal or a palladium compound and a copper compound by blowing (1,2) a feed gas mixture containing carbon monoxide and oxygen together with an inert gas or carbon dioxide into a reaction mixture (8) containing the liquid reactants, and passing an exhaust gas containing vapour formed by evaporation of liquid components of the reaction mixture through a condenser (11), the condensate being recirculated (12,13) to the reaction mixture and the remaining gas being discharged (14), characterised by blowing into the reaction mixture through a feed port (9) different from that for the feed gas mixture a gas selected from a portion of the discharged gas (14), an inert gas and carbon dioxide. This serves to increase the rate of evaporation of the liquid components of the reaction mixture to remove heat evolved in the reaction.

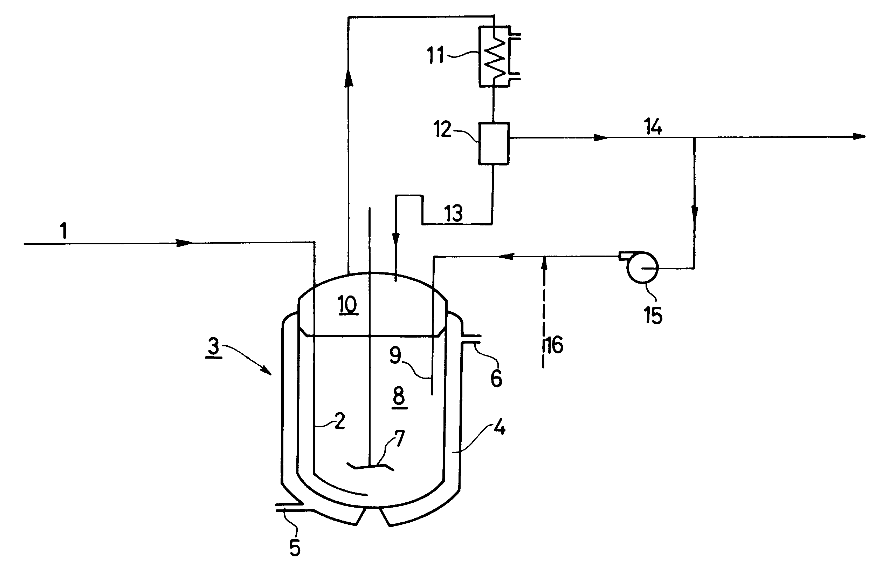

The accompanying sole drawing is a simplified flow chart showing a reactor employed in Examples of this invention.

As exemplary styrenes useful in the practice of this invention, may be mentioned styrene; alkyl derivatives of styrene such as α-methylstyrene, β-methylstyrene, α-ethylstyrene, β-ethylstyrene, o-methylstyrene, m-methylstyrene, p-methylstyrene, m-ethylstyrene, p-ethylstyrene, p-tert-butylstyrene and p-isopropyl-β-methylstyrene; and styrene derivatives containing on the aromatic ring one or more substituents which do not impair the reaction, such as p-chlorostyrene, p-methoxystyrene and 3,4-dimethoxystyrene.

Illustrative examples of the alcohol include aliphatic alcohols such as methanol, ethanol, propanol, butanol, pentanol, octanol, cyclopentanol and cyclohexanol. They may contain one or more substituents not impairing the reaction, such as halogen atoms and alkoxy groups.

Such an alcohol may be used in a proportion of 0.5-100 moles per mole of the styrene. The alcohol may be used not only as a starting material but also as a reaction solvent.

In the reaction according to the process of this invention, the starting alcohol may be used as a solvent in practice. One or more other solvents may also be used unless the reaction is impaired.

As palladium metal or its compound which is the first component of the catalyst employed in the process of this invention, may be mentioned by way of example palladium black; palladium metal carried on a carrier such as activated carbon, silica gel, alumina, silica-alumina, magnesia, zeolite or a molecular sieve; a 0-valent palladium complex such as palladium benzylideneacetone complex or tetrakis(triphenylphosphine) palladium; or a divallent palladium compound, for example, an inorganic acid salt of palladium such as palladium chloride or palladium nitrate, an organic acid salt of palladium such as palladium acetate or palladium benzoate, or a palladium complex such as bis(acetylacetonate) palladium, cyclooctadienedichloropalladium, palladium chloride benzonitrile complex, palladium chloride pyridine complex or palladium chloride amine complex. These palladium metals and palladium compounds may be used either singly or in combination.

Such a palladium metal or palladium compound may be used in a proportion of 0.1 gram atom or less, or preferably in a range of from 5 x 10⁻⁶ to 1 x 10⁻² gram atom per mole of the starting styrene.

As copper compound which is the second component of the catalyst employed in the process of this invention, may be mentioned by way of example a copper halide such as copper chloride or copper bromide; an inorganic acid salt of copper such as copper carbonate or copper nitrate; an organic acid salt of copper such as copper acetate, copper propionate, copper stearate, copper cinnamate or copper benzoate; or a copper complex compound such as copper acetyl acetonate or copper benzoyl acetonate. These copper compounds may be used either singly or in combination. Such a copper compound may preferably be soluble in the reaction mixture, but no problem or inconvenience will be encountered even if a portion of the copper compound remains undissolved. The copper compound may be used in a range of from 0.004 to 0.4 gram atom, preferably, from 0.008 to 0.3 gram atom per liter of the reaction mixture.

In the reaction according to the process of this invention, various other compounds may also be used without problem or inconvenience in order to improve the catalytic activities or reaction results or for other purposes. As illustrative examples of such compounds, may be mentioned (1) compounds of alkali metals, alkaline earth metals and aluminum-group metals; (2) compounds of metals selected from Group 4A, Group 5A, Group 7A, Group 8A (iron-group) and Group 2B of the periodic table; (3) compounds of rare earth elements; (4) halogen compounds; (5) inorganic and organic acids such as nitric acid and acetic acid; (6) tertiary amines; (7) nitriles; (8) dehydrating agents; (9) phenols and quinones. They may be used either singly or in combination.

In the reaction according to the process of this invention, gaseous starting materials are carbon monoxide and oxygen. They will hereinafter be called merely the "feed gas" when they are indicated collectively. In order to avoid the range of explosion, they are used as a mixture with an inert gas such as nitrogen or argon, or carbon dioxide. Air may be used as an oxygen source. It is preferable to use carbon dioxide in combination with the feed gas, because the existence of carbon dioxide in the reaction system can improve the reaction results. An inert gas and carbon dioxide may be used at the same time. In this invention, the above-mentioned inert gases and carbon dioxide will be called "inert gases or the like" collectively. In addition, a gaseous mixture of at least the feed gas and inert gases or the like, which are to be fed for the reaction, will be called the "feed gas mixture". The feed gas is usually fed as a single feed gas mixture for the reaction after mixing the feed gas in its entirety with the inert gases or the like. In some instances, the feed gas mixture may however be used by dividing it into two or more gases mixtures of different compositions. Although the feed gas mixture may always be prepared from fresh gases, a gas which has been taken out of the reaction system subsequent to its use in the reaction may be reused, as the feed gas mixture, for the next reaction after adjusting its composition as needed. This may be repeated. The feed gas mixture is fed by blowing it into the reaction mixture in the process of this invention. Its feeding may be effected through a single port or through plural ports. Various improvements are feasible in the process, including the installation of a spreader or diffuser with a view toward enhancing the spreading of the gas whenever needed.

In the process of this invention, the concentration range of the feed gas in the feed gas mixture, which is required to avoid the potential danger of explosion and to obtain satisfactory reaction results, is dependent on the kind of the inert gas or the like and reaction conditions, and cannot be specified. In general and preferably, except for other gases which may also be contained in the feed gas mixture in some instances, the concentrations of the individual feed gases in the gaseous mixture of carbon monoxide, oxygen, an inert gas or the like are 3-30 vol.% for carbon monoxide and 1.5-25 vol.% for oxygen. More preferably, the concentrations of carbon monoxide and oxygen range from 5 vol.% to 20 vol.% and from 3 vol.% to 18 vol.% respectively.

In the process of this invention, the ratio of carbon monoxide to oxygen in the feed gas mixture to be fed for the reaction is 0.5-3.0 as the molar ratio of carbon monoxide to oxygen. The reaction results or catalytic activities are reduced if their molar ratio falls below the lower limit or exceeds the upper limit, preferably, the molar ratio ranges from 0.8 to 2.2.

In terms of molar ratio to the starting styrene, the feed gas may be used in a range of from 0.5 to 5 in the case of carbon monoxide although this ratio varies depending on the amount of oxygen which is also contained at the same time. Molar ratios smaller than 0.5 result in remaining of the styrene unreacted in a large amount, so that the efficiency of the reaction is deteriorated. On the other hand, molar ratios greater than 5 leads to consumption of the feed gas in a much greater proportion for the formation of carbon dioxide through the direct reaction of carbon monoxide and oxygen than that consumed for the formation of the cinnamate ester, whereby the economy is deteriorated extremely. The preferable molar ratio is in a range of 1.0-4. On the other hand, the ratio range of oxygen to the styrene is automatically determined depending on the ratio range of carbon monoxide to the styrene and the above-described ratio range of carbon monoxide to oxygen.

The amounts of the individual feed gases to be used relative to the styrene can be expressed in terms of products of the concentrations of the respective feed gases in the feed gas mixture and the feed rate (flow rate) of the feed gas mixture when they are considered per unit time. Since both the concentrations and the amounts are limited to their respective ranges as described above, there is also a limitation to the range of the flow rate of the feed gas mixture and the flow rate of the feed gas mixture cannot hence be changed freely.

The partial pressure of carbon monoxide in the reaction according to the process of this invention is not higher than 5,000 kPa (50 atm) (abs. atm.; this definition will apply equally hereinafter), preferably, in a range of 0.5-4000 kPa (0.005-40 atm). The partial pressure of oxygen is not higher than 5,000 kPa (50 atm), preferably, in a range of (0.2-3,000 kPa) 0.002-30 atm. The overall pressure of the reaction is not higher than 50,000 kPa (500 atm), with 100-30,000 kPa (1-300 atm) being preferred.

The reaction temperature ranges from room temperature to 200°C, with 40°C-160°C being preferred.

No particular limitation is imposed on the reaction time, because it varies depending on reaction conditions. However, the reaction time may generally be 0.01-24 hours, preferably, 0.05-10 hours.

Under such conditions as described above, the feed gas mixture is blown at a certain flow rate into the reaction mixture. The gaseous mixture which has flowed out of the reactor is a mixed gas composed at least of carbon monoxide and oxygen remaining unconsumed in the reaction, the inert gas or the like amounting a major portion of the mixed gas, and vapor formed as a result of evaporation of liquid components in the reactor. The flow rate of the gaseous mixture increases as the flow rate of the gas passing through the reaction mixture becomes greater. The concentration of the vapor of the each liquid component in the gaseous mixture flowing out of the reactor is governed by the ratio of the vapor pressure of the liquid component, which is determined by the kind of the liquid component, its concentration in the reaction mixture and the temperature, to the overall pressure of the reaction. Accordingly, the flow rate of the vapor, in other words, the rate of evaporation is the product of the flow rate of the gaseous mixture flowing out of the reactor and the proportion of the vapor of the liquid components in the gaseous mixture. Here again, the rate of evaporation is dependent on the flow rate of the gas passing through the reaction mixture. As a consequence, the rate of evaporation increases as the flow rate of the gas increases. When the rate of evaporation is multiplied by the latent heat of vaporization of the liquid components under the given conditions, said latent heat of vaporization being an intrinsic value, the product is the removal rate of heat by the evaporation of the liquid components. This removal rate of heat is thus dependent on the flow rate of the gas passing through the reaction mixture. The removal rate of heat also increases as the flow rate of the gas increases. The gas passing through the reaction mixture is nothing but that derived from the feed gas mixture unless other gases are fed. Accordingly, the removal rate of heat depends on the flow rate of the feed gas mixture. As has already been described above, there is a suitable limiting range to the flow rate of the feed gas mixture. The flow rate of the feed gas mixture cannot hence be changed freely in order to achieve a necessary removal rate of heat.

The gaseous mixture, which has flowed out of the reactor, is caused to pass through a condenser, the resultant condensate is returned to the reactor, and the remaining gas is discharged. In order to remove heat from the reaction mixture at a necessary rate without deterioration of the reaction results and catalytic activities while maintaining the flow rate of the feed gas mixture within the preferable range, it is necessary to conduct the reaction by recirculating and blowing at least a portion of the thus-discharged gas into the reaction mixture through a feed port different from that for the feed gas mixture or as an alternative, blowing an inert gas such as nitrogen or argon, or carbon dioxide (inert gas or the like) provided separately into the reaction mixture through the different feed port. The different feed port may consists of a single opening or plural openings. As a result, the flow rate of the gas passing through the reaction mixture in the reactor can be increased so that the rate of evaporation of each liquid component can be increased. Neither the reaction results nor the catalytic activities are impaired by this practice. The flow rate of the discharged gas, which is recirculated, or the separately-provided inert gas or the like can be suitably chosen depending upon the necessary removal rate of heat. It is essential to blow the discharged recirculation gas or the separately-provided inert gas or the like into the reaction mixture through the port different from that for the feed gas mixture. If they should be fed through the same feed port, the gases of the different kinds are mixed in the piping so that the feed gas mixture is unnecessarily diluted or the ratio of carbon monoxide to oxygen in the feed gas mixture is changed away from the desired carbon monoxide/oxygen ratio, whereby certain deleterious effects may be given to the reaction results and catalytic activities. No substantial effects are expected for the enhancement of the removal rate of heat even if the discharged recirculation gas or the separately-provided inert gas or the like is fed into the vapor phase of the reactor instead of its blowing into the reaction mixture. Any improvements in the positional relationship, distance and configurations of the two types of feed ports for the feed gas mixture and the discharged recirculation gas or the separately-provided inert gas or the like, including the provision of one or more baffles at either one or both of the feed ports should be interpreted to fall within the scope of the concept of the present invention.

From the reaction mixture obtained as a result of the reaction by the process of this invention, the cinnamate ester can be isolated by a conventional method such as distillation.

According to the process of this invention, it is possible to achieve, with extreme ease, effective and economical removal of internal heat from a reactor upon preparation of a cinnamate ester by reacting its corresponding styrene and alcohol, carbon monoxide and oxygen in the presence of palladium metal or a compound thereof and a copper compound as catalysts, no matter how large the reaction scale is. The present invention therefore permits preparation of a cinnamate ester on an industrial scale.

Now, the present invention will be described in detail in reference to an accompanying drawing.

### Example 1

In a glass-lined 10-liter reactor 3 equipped with a stirrer 7 as shown in the drawing attached hereto were placed 0.39 g (2.2 millimoles) of palladium chloride, 19.0 g of cupric chloride, 84.7 g of cupric acetate monohydrate and 165 g of manganous acetate tetrahydrate, and about 1,000 g of methanol and 2,292 g (22.0 moles) of styrene were then added thereto. Afterward, methanol was added thereto so as to bring the total amount of the methanol into 3,525 g, whereby a reaction mixed liquid 8 was formed, and stirring was then commenced.

A feed gas mixture comprising carbon monoxide, oxygen and carbon dioxide in a ratio of 12:7:81 in terms of volume percent which had been regulated by a gas mixing machine was continuously blown into the reaction mixed liquid 8 through a line 1 and an introduction pipe 2 having an opening (feed gas inlet orifice) above the bottom of the reactor at a flow rate of 1.36 m³/hr (standard state; the same shall apply hereinafter), and temperature rise was commenced while the pressure in the reactor was maintained at a level of 850 kPa (8.5 atm).

The gas was discharged through a gaseous phase 10 in the reactor, a condenser 11, a gas-liquid separator 12 and an exhaust gas line 14. In this case, by the temperature reached to 100°C, a portion of the exhaust gas was circularly blown into the reaction mixed liquid at a flow rate of 2.74 m³/hr with the aid of a blower 15 from the branch of the exhaust line 14 through an introduction pipe 9 (exhaust gas circulation orifice) which had an opening at a position 65 cm under the surface of the reaction liquid. Reaction was continued at a reaction temperature of 100° C under a reaction pressure of 850 kPa (8.5 atm) for 6 hours, while the raw material mixed gas and the circulation exhaust gas were regulated so as to keep up the above-mentioned flow rates and while the liquid condensed in the condenser 11 was separated in the gas-liquid separator 12 and was caused to flow back to the reactor 3 through a condensed liquid circulation line 13.

During this period of time, pressurizing hot water was caused to flow through a jacket 4 for the reactor in order to maintain the reaction temperature (temperature of the reaction mixed liquid) at 100°C, and the temperature of the pressurizing hot water in the jacket 4 was 116°C at a jacket inlet 5 and 114°C at a jacket outlet 6 after a reaction time of 0.5 hour had elapsed, though it was perceptibly irregular at an early stage of the reaction. Afterward, the temperatures of the pressurizing hot water at these positions were slightly changed, and after 6 hours, they were 118°C at the inlet and 116°C at the outlet. During the reaction, the temperature at the jacket inlet was always higher than the temperature at the jacket outlet, and these temperatures were always higher than the above-mentioned reaction temperature. Thus, the jacket was a heating side. That is, this constitution permits sufficiently removing heat by the evaporation of liquid components in the reactor.

After the reaction, cooling and the release of pressure followed. The thus treated reaction liquid was analyzed by a high-speed liquid chromatograph, and it was confirmed that 1.08 moles of styrene and 18.94 moles of methyl cinnamate were contained therein. The conversion of styrene was 95.1%, the selectivity of methyl cinnamate (yield on the charged styrene) was 86.1%. The number of cinnamate formed per a gram atom of palladium as a catalytic component (hereinafter referred to as Pd turnover) was 8,610.

### Comparative Example 1

The same procedure as in Example 1 was repeated to perform the reaction with the exception that the exhaust gas was not circulated through the reaction mixed liquid. In order to maintain a reaction temperature at 100°C, the temperature of hot water flowing through the jacket was 96°C at the inlet and 98°C at the outlet of the jacket after a reaction time of 0.5 hour had elapsed, and afterward, the temperatures of the hot water at the inlet and outlet were slightly changed and they were 98°C and 99°C, respectively, after 6 hours. During the reaction, the temperature at the jacket outlet was always higher than the temperature at the jacket inlet, and these temperatures were always lower than the above-mentioned reaction temperature. Thus, in this constitution, the jacket was a cooling side.

As be apparent from the foregoing, if the exhaust gas is not circulated, the removal of heat generated in the reactor cannot be achieved sufficiently by the evaporation alone of liquid components, and it is additionally necessary to cool the reactor from the outside.

According to the analysis of the thus treated reaction liquid, the conversion of styrene was 96.2%, the selectivity of methyl cinnamate was 90.1%, the yield of methyl cinnamate was 86.7%, and the Pd turnover was 8,670. As indicated by these values, reaction results and catalytic activity were similar to those of Example 1 irrespectively of the circulation of the exhaust gas.

### Example 2

The same procedure as in Example 1 was repeated to perform the reaction with the exception that instead of the circulation of an exhaust gas, a carbon dioxide gas was blown into a reaction mixed liquid from the outside through a line 16 and the exhaust gas circulation orifice 9 used in Example 1 at the same flow rate as that of the exhaust gas in Example I. In order to maintain a reaction temperature at 100°C, temperatures of hot water at the inlet and outlet of the jacket were 114°C and 112°C, respectively, after a reaction time of 0.5 hour had elapsed. After 6 hours, temperatures thereof were 116°C and 114°C, respectively. That is, the temperatures in the jacket were about the same as in Example 1, and during this period of time, the jacket always was a heating side.

With regard to the results of the reaction, the conversion of styrene was 93.9%, and the selectivity of methyl cinnamate and the yield of methyl cinnamate were 91.2% and 85.6%, respectively. The Pd turnover was 8,560.

Also in this method, the removal velocity of heat generated in the reactor can be accelerated without impairing the reaction results and catalytic activity at all.

### Comparative Example 2

The same procedure as in Example 1 was repeated to perform the reaction with the exception that instead of blowing the circulation exhaust gas into the reaction mixed liquid, the exhaust gas was blown together with the feed gas mixture into the reaction mixed liquid by connecting the exhaust gas circulation line to the feed line 1, at the same flow rates as in Example 1. After 0.5 hour of the reaction, temperatures at the inlet and outlet of the jacket were 115°C and 113°C, respectively, and after 6 hours, they were 116°C and 114°C, respectively. That is, during this period of time, the jacket was always a heating side. With regard to the results of the reaction, the conversion of styrene was 81.6%, and the selectivity and the yield of methyl cinnamate were 86.4% and 70.5%, respectively. The Pd turnover was 7,050. In this example, the effect of accelerating the heat removal velocity was appreciated, but it was definite that the reaction was adversely affected.

### Comparative Example 3

In all the same manner as in Example 1, the reaction was commenced. After 2 hours, temperatures at the inlet and outlet of the jacket were 115°C and 113°C, respectively. While this condition was kept up, the blowing operation of the circulation exhaust gas into the reaction mixed liquid through the exhaust gas circulation orifice was switched to an operation by which the circulation exhaust gas was returned to the gaseous phase in the reactor. Immediately, the temperature of the reaction mixed liquid began to rapidly rise from 100°C, and it was steady at 117°C. As is apparent from the above, the circulation of the exhaust gas through the gaseous phase cannot provide any effect of accelerating the heat removal velocity.

## Claims

1. A process for preparing a cinnamate ester which comprises reacting the corresponding styrene compound with an alcohol, carbon monoxide and oxygen in the presence of a catalyst containing palladium metal or a palladium compound and a copper compound by blowing (1,2) a feed gas mixture containing carbon monoxide and oxygen together with an inert gas or carbon dioxide into a reaction mixture (8) containing the liquid reactants, and passing an exhaust gas containing vapour formed by evaporation of liquid components of the reaction mixture through a condenser (11), the condensate being recirculated (12,13) to the reaction mixture and the remaining gas being discharged (14), characterised by blowing into the reaction mixture through a feed port (9) different from that for the feed gas mixture a gas selected from a portion of the discharged gas (14), an inert gas and carbon dioxide (16).

2. A process as claimed in claim 1, wherein the feed gas mixture contains carbon dioxide.

3. A process as claimed in claim 1 or claim 2, wherein the styrene compound is styrene.

4. A process as claimed in any one of claims 1 to 3, wherein the gas blown into the reaction mixture through the different feed port is a portion of the discharged gas.

5. A process as claimed in any one of claims 1 to 3, wherein the gas blown into the reaction mixture through the different feed port is selected from inert gas and carbon dioxide.

## Patentansprüche

1. Verfahren zur Herstellung von Zimtsäureestern, das die Schritte umfaßt, eine entsprechende Styrol-Verbindung mit einem Alkohol, Kohlenmonoxid und Sauerstoff in Gegenwart eines Katalysators, der Palladium Metall oder eine Palladium-Verbindung und eine Kupfer-Verbindung enthält, dadurch umzusetzen, daß ein Einspeisgasgemisch, das Kohlenmonoxid und Sauerstoff zusammen mit einem Inertgas oder Kohlendioxid enthält, in ein Reaktionsgemisch (8), das die flüssigen Reaktanden enthält, eingeblasen (1,2) wird, und ein Abgas, das den durch die Verdampfung der flüssigen Bestandteile des Reaktionsgemisches gebildeten Dampf enthält, durch einen Kondensator (11) zu leiten, wobei das Kondensat in das Reaktionsgemisch zurückgeführt (12, 13) und das verbleibende Gas ausgetragen (14) wird, dadurch gekennzeichnet, daß ein Gas, ausgewählt aus einem Teil des ausgetragenen Gases (14), einem Inertgas und Kohlendioxid (16), über einen Einspeisstutzen (9), der verschieden ist von dem für das Einspeisgasgemisch, in das Reaktionsgemisch eingeblasen wird.

2. Das Verfahren nach Anspruch 1, wobei das Einspeisgasgemisch Kohlendioxid enthält.

3. Das Verfahren nach Anspruch 1 oder 2, wobei die Styrol-Verbindung Styrol ist.

4. Das Verfahren nach einem der vorhergehenden Ansprüche 1 bis 3, wobei das Gas, das durch den verschiedenen Einlaßstutzen in das Reaktionsgemisch geblasen wird, ein Teil des ausgetragenenen Gases ist.

5. Das Verfahren nach einem der vorhergehenden Ansprüche 1 bis 3, wobei das Gas, das durch den verschiedenen Einlaßstutzen in das Reaktionsgemisch geblasen wird, ausgewählt ist aus einem Inertgas und Kohlendioxid.

## Revendications

1. Procédé de préparation d'un ester cinnamique qui comprend de faire réagir le composé de styrène correspondant avec un alcool, du monoxyde de carbone et de l'oxygène en présence d'un catalyseur contenant du palladium métallique ou un composé de palladium et un composé de cuivre en insufflant (1,2) un mélange de gaz d'alimentation contenant du monoxyde de carbone et de l'oxygène avec un gaz inerte ou du dioxyde de carbone dans un mélange de réaction (8) contenant les réactants liquides, et en faisant passer un gaz d'évacuation contenant de la vapeur formée par l'évaporation des composants liquides du mélange de réaction à travers un condenseur (11), le condensat étant remis en circulation (12, 13) vers le mélange de réaction et le gaz restant étant évacué (14), caractérisé par l'insufflation dans le mélange de réaction par un orifice d'alimentation (9) différent de celui du mélange de gaz d'alimentation , d'un gaz choisi à partir d'une partie du gaz évacué (14), d'un gaz inerte et de dioxyde de carbone (16).

2. Procédé selon la revendication 1, dans lequel le mélange de gaz d'alimentation contient du dioxyde de carbone.

3. Procédé selon la revendication 1 ou 2, dans lequel le composé de styrène est du styrène.

4. Procédé selon l'une quelconque des revendications 1 à 3, dans lequel le gaz insufflé dans le mélange de réaction par l'orifice d'alimentation différent est une partie du gaz évacué.

5. Procédé selon l'une quelconque des revendications 1 à 3 , dans lequel le gaz insufflé dans le mélange de réaction par l'orifice d'alimentation différent est choisi à partir d'un gaz inerte et de dioxyde de carbone.
